# EUROPÄISCHE PATENTSCHRIFT

(11) **EP 2 931 382 B1**
(45) Veröffentlichungstag und Bekanntmachung des Hinweises auf die Patenterteilung: **27.06.2018**
(21) Anmeldenummer: 13792393.4
(22) Anmeldetag: 20.11.2013
(51) Int. Cl.: A61Q 19/02, A61K 8/35, A61K 8/97, A61K 36/31, A61K 36/33

(54) **WIRKSTOFFKOMBINATION ZUR HAUTAUFHELLUNG I**
ACTIVE SUBSTANCE COMBINATION FOR SKIN LIGHTENING I
COMBINAISON DE PRINCIPES ACTIFS POUR L'ÉCLAIRCISSEMENT DE LA PEAU I

(30) Priorität: 12.12.2012 DE 102012222970
(43) Veröffentlichungstag der Anmeldung: 21.10.2015
(73) Patentinhaber: Henkel AG & Co. KGaA, 40589 Düsseldorf (DE)
(72) Erfinder: KEßLER-BECKER, Daniela, 51371 Leverkusen (DE); DICKHOF, Susanne, 41748 Viersen (DE)
(86) Internationale Anmeldenummer: PCT/EP2013/074220
(87) Internationale Veröffentlichungsnummer: WO 2014/090526

(56) Entgegenhaltungen:
- EP-A1- 2 389 922
- EP-A2- 2 159 223
- WO-A1-2007/128723
- WO-A2-2006/137100
- WO-A2-2008/015342
- FR-A1- 2 820 036
- FR-A1- 2 869 228
- DATABASE GNPD [Online] MINTEL; 1. November 2006 (2006-11-01), Gatineau: "Skin renewing peeling kit", XP002730556, Database accession no. 610359
- DATABASE GNPD [Online] MINTEL; 1. November 2009 (2009-11-01), Patricia Wexler: "Calming Anti-Aging Moisturizer SPF 30", XP002730557, Database accession no. 1206135
- DATABASE GNPD [Online] MINTEL; 1. November 2010 (2010-11-01), DERMAdoctor: "Photo Dynamic Therapy SPF 30", XP002730558, Database accession no. 1442681
- KURILICH A C ET AL: "Carotene, Tocopherol, Ascorbate Contents in Subspecies of Brassica oleracea", JOURNAL OF AGRICULTURAL AND FOOD CHEMISTRY, AMERICAN CHEMICAL SOCIETY, US, Bd. 47, Nr. 4, 1. Januar 1999 (1999-01-01) , Seiten 1576-1581, XP002980144, ISSN: 0021-8561, DOI: 10.1021/JF9810158
- BUTERA D ET AL: "Antioxidant Activities of Sicilian Prickly Pear (Opuntia ficus-indica), Fruit Extracts, and Reducing Properties of its Betalains: Betanin and Indicaxanthin", JOURNAL OF AGRICULTURAL AND FOOD CHEMISTRY, AMERICAN CHEMICAL SOCIETY, US, Bd. 50, Nr. 23, 1. Januar 2002 (2002-01-01), Seiten 6895-6901, XP002993966, ISSN: 0021-8561, DOI: 10.1021/JF025696P

## Beschreibung

Die vorliegende Erfindung betrifft kosmetische oder dermatologische Zusammensetzungen zur kosmetischen und topischen dermatologischen Hautaufhellung oder zur Verhinderung der Hautbräunung, insbesondere der durch UV-Strahlung hervorgerufenen Hautbräunung, sowie zur Aufhellung von Altersflecken oder Sommersprossen.
Die Pigmentierung der Haut erfolgt durch Melanozyten, welche in der untersten Schicht der Epidermis neben den Basalzellen als je nach Hauttyp entweder vereinzelt oder gehäuft auftretende pigmentbildende Zellen vorzufinden sind. Melanozyten enthalten Melanosomen, in denen Melanin gebildet wird. Durch verschiedene chemische und/oder physikalische Einflüsse, insbesondere durch UV-Strahlung, wird verstärkt Melanin gebildet. Dieses wird über die Keratinozyten in die Corneozyten (Hornschicht) transportiert und führt zu einer bräunlichen bis braun-schwarzen Hautfarbe. Melanin wird als Endstufe eines oxidativen Prozesses gebildet, in welchem Tyrosin unter Mitwirkung der Enzyms Tyrosinase über mehrere Zwischenstufen zu den braun bis braunschwarzen Eumelaninen (DHICA- und DHI-Melanin) bzw. unter Beteiligung von schwefelhaltigen Verbindungen zum rötlichen Phäomelanin umgewandelt wird. Die Melaninbildung - und damit Hautfarbe - unterliegt äußeren Einflüssen und kann neben erwünschten Effekten ("gesunde Bräune") auch zu unerwünschten Erscheinungen führen. So kann z.B. UV-Strahlung zu Sommersprossen führen. Fehlpigmentierungen können auch aufgrund genetischer Disposition, Wundheilung bzw. -vernarbung oder Hautalterung ("Altersflecken") auftreten. Da der Prozess der Desquamation (oberflächliche Loslösung von Zellen oder Zellgruppen aus ihrem epithelialen Verband) einen beständigen Verlust an Melanin beinhaltet, kann eine Aufhellung der Haut durch Inhibierung der Neusynthese von Melanin erreicht werden. Altersflecken stellen ein Zeichen der Hautalterung dar, das einer speziellen Behandlung bedarf. Da es sich nicht um eine intensivere Pigmentierung der Haut, sondern konkret um Anhäufungen des bräunlich-wachsartigen Pigments Lipofuscin (auch Alters- oder Abnutzungspigment), das als Endprodukt aus der Oxidation von ungesättigten Fettsäuren der Zellmembranen entsteht. Die Lysosomen sind nicht mehr imstande, den Stoff vollständig abzubauen. So bleibt er als Fleck zurück.
Wirksame kosmetische Zusammensetzungen zur Hautaufhellung sind bekannt. Diese enthalten als Wirkstoff jedoch zumeist Hydrochinon oder Kojisäure (zur Inhibierung der Tyrosinase). Beide Substanzen sind mutagen und sollten daher nicht über längere Zeiträume verwendet werden.

Auch alternative Wirkstoffe zur Hautaufhellung sind im Stand der Technik offenbart. So wird unter der Bezeichnung Skin Moon® von der Firma Bionap eine Mischung aus Pflanzenextrakten vertrieben, welche Extrakte aus Opuntia Ficus Indica enthält und sich zur Hautaufhellung auch unter Sonneneinwirkung eignet. Entsprechende Wirkstoffe sind unter der Bezeichnung EXFOLACTIVE® auch von der Firma Silab erhältlich. Extrakte aus Opuntia Ficus Indica stimulieren die Aktivität von Hautenzymen, welche in den Exfoliationsprozeß involviert sind.

Fermentierte und hydrolisierte Proteine aus Brassicae-Pflanzen, insbesondere aus Brassica Napus werden von der Firma ISP unter der Bezeichnung Achromaxyl® als besonders wirksame hautaufhellende Mittel vertrieben.

Es bestand nach wie vor die Aufgabe, lagerstabile hautaufhellende Zusammensetzungen zur Verfügung zu stellen, die hochwirksam, auch unter UV-Bestrahlung (Sonnenlicht) anwendbar und möglichst auf Naturstoffen basiert sind. Darüber hinaus sollten weitere positive Effekte auf älterer Haut ermöglicht werden, insbesondere eine Reduzierung von Falten.

Überraschender Weise wurde nun gefunden, daß eine Kombination zweier bekannter Hautaufhellungsmittel mit einem bestimmten UV-Schutz besonders gute hautaufhellende Eigenschaften zeigt, die auch unter UV-Bestrahlung (Sonnenlicht) nutzbar sind.

Gegenstand der vorliegenden Erfindung sind in einer ersten Ausführungsform kosmetische Mittel zur Aufhellung der Haut und/oder von Altersflecken, enthaltend
a) mindestens einen Extrakt aus Opuntia Ficus Indica;
b) mindestens einen Extrakt aus Brassicae-Pflanzen;
c) 2,3-Dihydro-5,6-dimethoxy-3,3-dimethyl-2-(phenylmethylen)-1H-Inden-1-on (Benzyliden Dimethoxydimethylindanon).

Als ersten wesentlichen Inhaltsstoff enthalten die erfindungsgemäßen Mittel mindestens einen Extrakt aus Opuntia Ficus Indica. Opuntia ficus-indica ist eine Pflanzenart in der Gattung der Opuntien (*Opuntia*) aus der Familie der Kakteengewächse (Cactaceae). Das Artepitheton *ficus-indica* bedeutet ,indische Feige'. Extrakte aus Opuntien können nach bekannten Herstellungsverfahren in variabler Zusammensetzung mit Lösungsmitteln wie z. B. Wasser, Methanol, Ethanol, Aceton, etc., und deren Gemische bei Temperaturen von Raumtemperatur bis 100 °C unter gelinder bis heftiger Durchmischung innerhalb von 10 Min. bis 24 Std. unter Normaldruck bis zu 200 bar erhalten werden. Zur Anreicherung wirksamkeitsbestimmender Komponenten können weitere Konzentrierungsschritte durchgeführt werden wie z. B. flüssig-flüssig-Verteilung mit z. B. 1-Butanol/Wasser oder Ethylacetat/Wasser, Adsorption-Desorption an Ionenaustauscher, LH20, HP20 und andere Harze oder chromatographische Abtrennungen über RP18, Kieselgel, etc.. Falls die Weiterverarbeitung zu Trockenextrakten erwünscht ist, erfolgt diese nach an sich bekannten Verfahren durch Abziehen des Lösungsmittels bei erhöhter Temperatur und / oder reduziertem Druck.

Erfindungsgemäß bevorzugte kosmetische Mittel sind dadurch gekennzeichnet, daß sie, bezogen auf das Gewicht des anwendungsbereiten Mittels 0,001 bis 20 Gew.-%, vorzugsweise 0,005 bis 15 Gew.-%, weiter bevorzugt 0,01 bis 10 Gew.-%, noch weiter bevorzugt 0,05 bis 5 Gew.-% und insbesondere 0,1 bis 1 Gew.-% mindestens eines Extraktes aus Opuntia Ficus Indica enthalten.

Die durch Extraktion gewonnenen Inhaltsstoffe können chemisch modifiziert werden, wobei insbesondere der Hydrolyse eine besondere Bedeutung zukommt. Erfindungsgemäße kosmetische Mittel, die, bezogen auf das Gewicht des anwendungsbereiten Mittels 0,001 bis 20 Gew.-%, vorzugsweise 0,005 bis 15 Gew.-%, weiter bevorzugt 0,01 bis 10 Gew.-%, noch weiter bevorzugt 0,05 bis 5 Gew.-% und insbesondere 0,1 bis 1 Gew.-% mindestens eines ganz oder teilweise hydrolysierten Extraktes aus Opuntia Ficus Indica (CAS-Nr. 90082-21-6) enthalten, sind bevorzugte Ausführungsformen der vorliegenden Erfindung.

Entsprechende Extrakte aus Opuntia Ficus Indica sind kommerziell erhältlich, beispielsweise unter der Bezeichnung Skin Moon® von der Firma Bionap oder EXFOLACTIVE® von der Firma Silab.

Als zweiten wesentlichen Inhaltsstoff enthalten die erfindungsgemäßen Mittel mindestens einen Extrakt aus Brassicae-Pflanzen. Die Kreuzblütengewächse (Brassicaceae oder Cruciferae), auch Kreuzblütler genannt, sind eine Pflanzenfamilie in der Ordnung der Kreuzblütlerartigen (Brassicales). Die Familie enthält etwa 336 (bis 419) Gattungen mit etwa 3.000 bis 4.130 Arten. Sie ist durch viele Kulturpflanzen von großer wirtschaftlicher Bedeutung.

Erfindungsgemäß bevorzugte kosmetische Mittel sind dadurch gekennzeichnet, daß sie, bezogen auf das Gewicht des anwendungsbereiten Mittels 0,001 bis 20 Gew.-%, vorzugsweise 0,01 bis 17,5 Gew.-%, weiter bevorzugt 0,1 bis 15 Gew.-%, noch weiter bevorzugt 0,5 bis 10 Gew.-% und insbesondere 1 bis 5 Gew.-% mindestens eines Extraktes aus Brassicae-Pflanzen enthalten.

Unter den Brassica-Pflanzen hat sich insbesondere Brassica Napus als besonders geeignet erwiesen. Extrakte aus Brassica Napus steigern die hautaufhellende Wirkung der Opuntien-Extrakte und führen überdies zu einer verbesserten Hautverträglichkeit und zu einer intensiveren und reizärmeren Wirkung auch unter Sonneneinwirkung.

Raps (*Brassica napus*) ist eine Pflanzenart aus der Familie der Kreuzblütengewächse (Brassicaceae). Die Steckrübe *Brassica napus* subsp. *rapifera* ist eine Unterart von Raps (*Brassica napus*).

Erfindungsgemäße kosmetische Mittel, die, bezogen auf das Gewicht des anwendungsbereiten Mittels 0,001 bis 20 Gew.-%, vorzugsweise 0,01 bis 17,5 Gew.-%, weiter bevorzugt 0,1 bis 15 Gew.-%, noch weiter bevorzugt 0,5 bis 10 Gew.-% und insbesondere 1 bis 5 Gew.-% mindestens eines Extraktes aus Brassica Napus enthalten, sind bevorzugte Ausführungsformen der vorliegenden Erfindung.

Auch beim zweiten Extrakt können die durch Extraktion gewonnenen Inhaltsstoffe chemisch modifiziert werden, wobei insbesondere der Hydrolyse eine besondere Bedeutung zukommt. Es hat sich darüber hinaus als vorteilhaft erwiesen, Pflanzenteile von Braissica Napus vor der Extraktion zu fermentieren, da die Wirksamkeit der Extrakte auf diese Weise gesteigert wird. Erfindungsgemäße kosmetische Mittel, die, bezogen auf das Gewicht des anwendungsbereiten Mittels 0,001 bis 20 Gew.-%, vorzugsweise 0,01 bis 17,5 Gew.-%, weiter bevorzugt 0,1 bis 15 Gew.-%, noch weiter bevorzugt 0,5 bis 10 Gew.-% und insbesondere 1 bis 5 Gew.-% mindestens eines ganz oder teilweise hydrolysierten Extraktes aus fermentierten Pflanzenteilen von Brassica Napus enthalten, sind besonders bevorzugt.

Vorzugsweise werden die beiden Extrakte a) und b) in einem bestimmten Verhältnis zueinander eingesetzt, wobei es sich als vorteilhaft erwiesen hat, wenn die Brassicae-Extrakte in höheren mengen eingesetzt werden als die Opuntien-Extrakte. Besonders bevorzugte erfindungsgemäße kosmetische Mittel sind dadurch gekennzeichnet, daß das Gewichtsverhältnis von b) zu a) > 1 zu 1, vorzugsweise > 1,1 zu 1, weiter bevorzugt > 1,25 zu 1 und insbesondere > 1,5 zu 1 beträgt.

Als dritten wesentlichen Inhaltsstoff enthalten die erfindungsgemäßen Mittel 2,3-Dihydro-5,6-dimethoxy-3,3-dimethyl-2-(phenylmethylen)-1H-Inden-1-on (Benzyliden Dimethoxydimethylindanon). Dieser Wirkstoff schützt die Haut vor schädlichen Einwirkungen der Umwelt wie Zigarettenrauch, Autoabgasen und UVB-Strahlen. Die essentielle Aminosäure Tryptophan bildet aufgrund von UVB-Einstrahlungen eine toxische Substanz. Koppelt sich diese Substanz an das Transmitter-Protein Aryl hydrocarbon Receptor (AhR), können Gifte bis tief in die Zellen gelangen und dort Alterungsprozesse beschleunigen.
2,3-Dihydro-5,6-dimethoxy-3,3-dimethyl-2-(phenylmethylen)-1H-Inden-1-on bindet das Protein AhR und verhindert damit die Kopplung der schädigenden Substanzen.

Erfindungsgemäß bevorzugte kosmetisches Mittel sind dadurch gekennzeichnet, daß sie, bezogen auf das Gewicht des anwendungsbereiten Mittels 0,001 bis 20 Gew.-%, vorzugsweise 0,01 bis 17,5 Gew.-%, weiter bevorzugt 0,1 bis 15 Gew.-%, noch weiter bevorzugt 0,5 bis 10 Gew.-% und insbesondere 1 bis 5 Gew.-% 2,3-Dihydro-5,6-dimethoxy-3,3-dimethyl-2-(phenylmethylen)-1H-Inden-1-on (Benzyliden Dimethoxydimethylindanon) enthalten.

Die erfindungsgemäßen Mittel enthalten die jeweiligen Wirkstoffe in einem kosmetisch verträglichen Träger. Dieser kosmetische Träger ist bevorzugt wässrig, alkoholisch, wässrig-alkoholisch oder wässrig-glycolisch. Solche Träger sind beispielsweise Cremes, Emulsionen, Gele oder auch tensidhaltige schäumende Lösungen, wie beispielsweise Duschgele oder andere Zubereitungen, die für die Anwendung auf der Haut geeignet sind. Ein wässriger Träger enthält im Sinne der Erfindung bevorzugt mindestens 40 Gew.-%, besonders bevorzugt mindestens 50 Gew.-% Wasser. Erfindungsgemäß bevorzugte kosmetische Mittel sind dadurch gekennzeichnet, daß sie, bezogen auf das Gewicht des anwendungsbereiten Mittels 10 bis 70 Gew.-%, vorzugsweise 20 bis 65 Gew.-%, weiter bevorzugt 30 bis 60 Gew.-%, noch weiter bevorzugt 35 bis 55 Gew.-% und insbesondere 40 bis 50 Gew.-% Wasser enthalten.

Unter wässrig-alkoholischen Trägern sind im Sinne der vorliegenden Erfindung wasserhaltige Zusammensetzungen, enthaltend 3 bis 70 Gew.-%, bevorzugt 10 - 60 Gew.-%, besonders bevorzugt 15 - 40 Gew.-%, eines einwertigen C₂-C₄-Alkohols, insbesondere Ethanol, zu verstehen.
Unter wässrig-glycolischen Trägern sind im Sinne der vorliegenden Erfindung wasserhaltige Zusammensetzungen, enthaltend 3 bis 70 Gew.-%, bevorzugt 10 - 60 Gew.-%, besonders bevorzugt 15 - 40 Gew.-%, mindestens eines zwei- oder mehrwertigen C₂-C₉-Alkohols oder mindestens eines wasserlöslichen Polyethylenglycols mit 3 - 20 Ethylenoxid-Einheiten, insbesondere ausgewählt aus 1,2-Propylenglycol, 2-Methyl-1,3-propandiol, Glycerin, 1,2-Butylenglycol, 1,3-Butylenglycol, 1,4-Butylenglycol, Pentylenglycolen wie 1,2-Pentandiol und 1,5-Pentandiol, Hexandiolen wie 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, PEG-3, PEG-4, PEG-6, PEG-7, PEG-8, PEG-9, PEG-10, PEG-12, PEG-14, PEG-16, PEG-18 und PEG-20 sowie Mischungen hiervon, zu verstehen.
Die erfindungsgemäßen Mittel können zusätzlich weitere organische Lösemittel, wie beispielsweise Methoxybutanol, Ethyldiglycol, 1,2-Propylenglycol, 2-Methyl-1,3-propandiol, n-Propanol, n-Butanol, n-Butylenglycol, Glycerin, Diethylenglycolmonoethylether und Diethylenglycolmono-n-butylether, enthalten. Bevorzugt sind dabei alle wasserlöslichen organischen Lösemittel.

Die erfindungsgemäßen Zusammensetzungen können in allen für die topische Applikation auf die Haut geeigneten Darreichungsformen konfektioniert sein. Bevorzugte Darreichungsformen sind eine Lösung, eine Emulsion vom Typ Wasser-in-ÖI (W/O), eine Emulsion vom Typ Öl-in-Wasser (O/W), eine multiple Emulsion, beispielsweise vom Typ Wasser-in-ÖI-in-Wasser (W/O/W) oder Öl-in-Wasser-in-ÖI (O/W/O), eine Hydrodispersion, eine Lipodispersion, ein Gel, ein Hydrodispersionsgel, ein Lipodispersionsgel, ein fester Stift, ein Gelpflaster (Gelpatch), ein Pflaster, ein Kataplasma, Wirkstoffhaltige Liposomen oder ein transdermales therapeutisches System. Niedrig-viskose oder mittelviskose Darreichungsformen können auch in einem treibgasfreien Pump- oder Sprühspender oder zusammen mit einem Treibmittel in einem Aerosolbehälter konfektioniert sein.
Die anwendungsbereiten erfindungsgemäßen Mittel können weitere Hilfs- und Zusatzstoffe enthalten. So hat es sich für die Applikation und den Verbleib des Mittels auf der Haut als vorteilhaft erwiesen, wenn die Zusammensetzungen mindestens ein Verdickungsmittel enthalten. Bezüglich dieser Verdickungsmittel bestehen keine prinzipiellen Einschränkungen. Es können sowohl organische als auch rein anorganische Verdickungsmittel zum Einsatz kommen. Geeignete Verdickungsmittel sind
- anionische, synthetische Polymere;
- kationische, synthetische Polymere;
- natürlich vorkommende Verdickungsmittel, wie Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, sowie Cellulosederivate, wie beispielsweise Methylcellulose, Carboxyalkylcellulosen und Hydroxyalkylcellulosen;
- nichtionische, vollsynthetische Polymere, wie Polyvinylalkohol oder Polyvinylpyrrolidinon; sowie
- anorganische Verdickungsmittel, insbesondere Schichtsilikate wie beispielsweise Bentonit, besonders Smektite, wie Montmorillonit oder Hectorit.
Vorzugsweise werden die Mittel als fließfähige Zubereitungen bereitgestellt. "Fließfähig" im Sinne der vorliegenden Anmeldung sind dabei Zusammensetzungen, die gießbar sind und Viskositäten von 10 mPa·s bis hin zu 250.000 mPa·s (20 °C) aufweisen können. Die Viskosität kann mit üblichen Standardmethoden (beispielsweise Brookfield-Viskosimeter LVT-II bei 20 U/min und 20°C, Spindel 3) gemessen werden und liegt vorzugsweise im Bereich von 50 bis 50000 mPa·s. Bevorzugte Mittel haben Viskositäten von 1000 bis 30000 mPas, wobei Werte von 5000 bis 20000 mPas besonders bevorzugt sind.
Die erfindungsgemäßen Mittel können weiterhin zusätzliche Wirk-, Hilfs- und Zusatzstoffe enthalten.

Erfindungsgemäße Emulsionen im Sinne der vorliegenden Erfindung, z. B. in Form einer Creme, einer Lotion oder einer kosmetischen Milch, sind vorteilhaft und enthalten z. B. Fette, Öle, Wachse und/oder andere Fettkörper, sowie Wasser und einen oder mehrere Emulgatoren, wie sie üblicherweise für einen solchen Typ der Formulierung verwendet werden.
Es ist auch möglich und vorteilhaft im Sinne der vorliegenden Erfindung, die erfindungsgemäßen Zusammensetzungen als wässrige Systeme bzw. Tensidzusammensetzungen zur Reinigung und Pflege der Haut bereitzustellen. Dies umfasst bevorzugt Duschgels, Kopfhauttonics, Sprays etc.

Als besonders vorteilhaft hat sich der Einsatz von Tensiden (E) in den erfindungsgemäßen Mitteln erwiesen. In einer weiteren bevorzugten Ausführungsform enthalten die erfindungsgemäßen Mittel daher Tenside. Unter dem Begriff Tenside werden grenzflächenaktive Substanzen, die an Ober- und Grenzflächen Adsorptionsschichten bilden oder in Volumenphasen zu Mizellkolloiden oder lyotropen Mesophasen aggregieren können, verstanden. Man unterscheidet Aniontenside bestehend aus einem hydrophoben Rest und einer negativ geladenen hydrophilen Kopfgruppe, amphotere Tenside, welche sowohl eine negative als auch eine kompensierende positive Ladung tragen, kationische Tenside, welche neben einem hydrophoben Rest eine positiv geladene hydrophile Gruppe aufweisen, und nichtionische Tenside, welche keine Ladungen sondern starke Dipolmomente aufweisen und in wässriger Lösung stark hydratisiert sind.
Als anionische Tenside (E1) eignen sich in erfindungsgemäßen Zusammensetzungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- sulfatierte Hydroxyalkylpolyethylen- und/oder Hydroxyalkylenpropylenglykolether
- Sulfonate ungesättigter Fettsäuren mit 8 bis 24 C-Atomen und 1 bis 6 Doppelbindungen
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (E1-I),

   R¹(OCH₂CH₂)ₙ-O-P(O)(OX)-OR² (E1-I)

   in der R¹ bevorzugt für einen aliphatischen Kohlenwasserstoffrest mit 8 bis 30 Kohlenstoffatomen, R² für Wasserstoff, einen Rest (CH₂CH₂O)ₙR² oder X, n für Zahlen von 1 bis 10 und X für Wasserstoff, ein Alkali- oder Erdalkalimetall oder NR³R⁴R⁵R⁶, mit R³ bis R⁶ unabhängig voneinander stehend für Wasserstoff oder einen C₁ bis C₄ - Kohlenwasserstoffrest, steht,
- sulfatierte Fettsäurealkylenglykolester der Formel (E1-II)

   R⁷CO(AlkO)ₙSO₃M (E1-II)

   in der R⁷CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht,
- Monoglyceridsulfate und Monoglyceridethersulfate der Formel (E1-III) 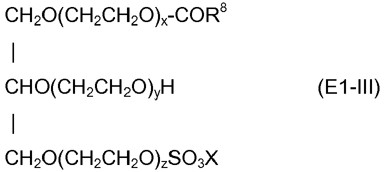 in der R⁸CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (E1-III) eingesetzt, in der R⁸CO für einen linearen Acylrest mit 8 bis 18 Kohlenstoffatomen steht,
- Amidethercarbonsäuren,
- Kondensationsprodukte aus C₈ - C₃₀ - Fettalkoholen mit Proteinhydrolysaten und/oder Aminosäuren und deren Derivaten, welche dem Fachmann als Eiweissfettsäurekondensate bekannt sind, wie beispielsweise die Lamepon® - Typen, Gluadin® - Typen, Hostapon® KCG oder die Amisoft® - Typen.
Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Monoglycerdisulfate, Alkyl- und Alkenyletherphosphate sowie Eiweißfettsäurekondensate.
Als zwitterionische Tenside (E2) werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ - Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.
Unter ampholytischen Tensiden (E3) werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ - Acylsarcosin. Nichtionische Tenside (E4) enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol® LS, Dehydol® LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen® HSP (Cognis) oder Sovermol - Typen (Cognis),
- alkoxylierte Triglyceride,
- alkoxylierte Fettsäurealkylester der Formel (E4-I)

   R¹CO-(OCH₂CHR²)_{w}OR³ (E4-I)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R⁴O-[G]ₚ (E4-II)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden.
Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (E4-II) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muss und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R⁴ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹⁵ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.
- Zuckertenside vom Typ der Fettsäure-N-alkylpolyhydroxyalkylamide, ein nichtionisches Tensid der Formel (E4-III),

   R⁵CO-NR⁶-[Z] (E4-III)

   in der R⁵CO für einen aliphatischen Acylrest mit 6 bis 22 Kohlenstoffatomen, R⁶ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden. Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher Fettsäure-N-alkylglucamide dar, wie sie durch die Formel (E4-IV) wiedergegeben werden:

   R⁷CO-NR⁸-CH₂-(CHOH)₄-CH₂OH (E4-IV)

   Vorzugsweise werden als Fettsäure-N-alkylpolyhydroxyalkylamide Glucamide der Formel (E4-IV) eingesetzt, in der R⁸ für Wasserstoff oder eine Alkylgruppe steht und R⁷CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkylglucamide der Formel (E4-IV), die durch reduktive Aminierung von Glucose mit Methylamin und anschließende Acylierung mit Laurinsäure oder C12/14-Kokosfettsäure bzw. einem entsprechenden Derivat erhalten werden. Weiterhin können sich die Polyhydroxyalkylamide auch von Maltose und Palatinose ableiten.
Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zusammensetzungen mit hervorragenden Eigenschaften werden auch erhalten, wenn sie als nichtionische Tenside Fettsäureester von ethoxyliertem Glycerin enthalten.
Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.
Weiterhin sind ganz besonders bevorzugte nichtionische Tenside die Zuckertenside. Diese können in den erfindungsgemäß verwendeten Mitteln bevorzugt in Mengen von 0,1 - 20 Gew.-%, bezogen auf das gesamte Mittel, enthalten sein. Mengen von 0,5 - 15 Gew.-% sind bevorzugt, und ganz besonders bevorzugt sind Mengen von 0,5 - 7,5 Gew.-%.
Erfindungsgemäß einsetzbar sind kationische Tenside vom Typ der quarternären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.
Bevorzugt einsetzbar sind erfindungsgemäß QAV mit Behenylresten, insbesondere die unter der Bezeichnung Behentrimoniumchlorid bzw. -bromid (Docosanyltrimethylammonium Chlorid bzw. -Bromid) bekannten Substanzen. Andere bevorzugte QAV weisen mindestens zwei Behenylreste auf, wobei QAV, welche zwei Behenylreste an einem Imidazoliniumrückgrat besonders bevorzugt sind. Kommerziell erhältlich sind diese Substanzen beispielsweise unter den Bezeichnungen Genamin® KDMP (Clariant) und Crodazosoft® DBQ (Crodauza).
Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethylammoniumchlorid, sowie Dehyquart® F-75, Dehyquart® C-4046, Dehyquart® L80 und Dehyquart® AU-35 sind Beispiele für solche Esterquats.
Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyl-dimethylamin dar.
Die kationischen Tenside sind in den erfindungsgemäßen Mitteln bevorzugt in Mengen von 0,05 bis 10 Gew.-%, bezogen auf das gesamte Mittel, enthalten. Mengen von 0,1 bis 5 Gew.-% sind besonders bevorzugt.
Die Tenside (E) werden vorzugsweise in Mengen von 0,1 - 45 Gew.%, bevorzugt 0,5 - 30 Gew.% und ganz besonders bevorzugt von 0,5 - 25 Gew.%, bezogen auf das gesamte erfindungsgemäße Mittel, eingesetzt.
Anionische, nichtionische, zwitterionische und/oder amphotere Tenside sowie deren Mischungen können erfindungsgemäß bevorzugt sein.
Zusammenfassend sind erfindungsgemäße kosmetische oder dermatologische Zusammensetzungen bevorzugt, die - bezogen auf ihr Gewicht - 0,5 bis 70 Gew.-%, vorzugsweise 1 bis 60 Gew.-% und insbesondere 5 bis 25 Gew.-% anionische(s) und/oder nichtionische(s) und/oder kationische(s) und/oder amphotere(s) Tensid(e), enthalten.
In einer weiteren bevorzugten Ausführungsform können die erfindungsgemäßen Mittel Emulgatoren (F) enthalten. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Erfindungsgemäß verwendbare Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov®68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phosphatidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls® PGPH),
- Lineare und verzweigte Fettsäuren mit 8 bis 30 C - Atomen und deren Na-, K-, Ammonium-, Ca-, Mg- und Zn - Salze.
Die erfindungsgemäßen Mittel enthalten die Emulgatoren bevorzugt in Mengen von 0,1 - 25 Gew.-%, insbesondere 0,5 - 15 Gew.-%, bezogen auf das gesamte Mittel.
Bevorzugt können die erfindungsgemäßen Zusammensetzungen mindestens einen nichtionogenen Emulgator mit einem HLB-Wert von 8 bis 18 enthalten. Nichtionogene Emulgatoren mit einem HLB-Wert von 10 - 15 können erfindungsgemäß besonders bevorzugt sein.
Als weiterhin vorteilhaft hat es sich gezeigt, wenn Polymere (G) in den erfindungsgemäßen Mitteln enthalten sind. In einer bevorzugten Ausführungsform werden den erfindungsgemäßen Mitteln daher Polymere zugesetzt, wobei sich sowohl kationische, anionische, amphotere als auch nichtionische Polymere als wirksam erwiesen haben.
Erfindungsgemäß einsetzbar sind vorzugsweise kationische bzw. amphotere Polymere. Unter kationischen bzw. amphoteren Polymeren sind Polymere zu verstehen, welche in der Haupt- und/ oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C1-4-Kohlenwasserstoffgruppe an eine aus Acrylsäure, Methacrylsäure oder deren Derivaten aufgebaute Polymerhauptkette gebunden sind, haben sich als besonders geeignet erwiesen.
Homopolymere der allgemeinen Formel (G1-I), 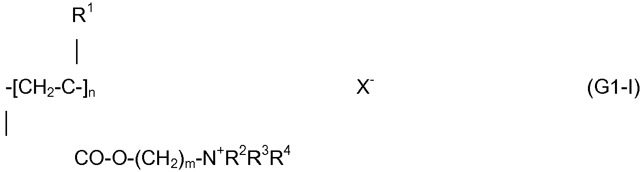 in der R¹= -H oder -CH₃ ist, R², R³ und R⁴ unabhängig voneinander ausgewählt sind aus C1-4-Alkyl-, - Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (G1-I) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt: R¹ steht für eine Methylgruppe, R², R³ und R⁴ stehen für Methylgruppen, m hat den Wert 2. Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.
Ein besonders geeignetes Homopolymer ist das, gewünschtenfalls vernetzte, Poly(methacryloyloxyethyltrimethylammoniumchlorid) mit der INCI-Bezeichnung Polyquaternium-37. Solche Produkte sind beispielsweise unter den Bezeichnungen Rheocare® CTH (Cosmetic Rheologies) und Synthalen® CR (Ethnichem) im Handel erhältlich. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.
Das Homopolymer wird bevorzugt in Form einer nichtwässrigen Polymerdispersion, die einen Polymeranteil nicht unter 30 Gew.-% aufweisen sollte, eingesetzt. Solche Polymerdispersionen sind unter den Bezeichnungen Salcare® SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare® SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich. Copolymere mit Monomereinheiten gemäß Formel (G1-I) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwässrige Polymerdispersion unter der Bezeichnung Salcare® SC 92 erhältlich.
Weitere bevorzugte kationische Polymere sind beispielsweise
- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate,
- kationische Alkylpolyglycoside gemäß der DE-PS 44 13 686,
- kationisierter Honig, beispielsweise das Handelsprodukt Honeyquat® 50,
- kationische Guar-Derivate, wie insbesondere die unter den Handelsnamen Cosmedia®Guar und Jaguar® vertriebenen Produkte,
- polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und - methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat®734 und Gafquat®755 im Handel erhältlich,
- Vinylpyrrolidon-Vinylimidazoliummethochlorid-Copolymere, wie sie unter den Bezeichnungen Luviquat® FC 370, FC 550, FC 905 und HM 552 angeboten werden,
- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette.
Gleichfalls als kationische Polymere eingesetzt werden können die unter den Bezeichnungen Polyquaternium-24 (Handelsprodukt z. B. Quatrisoft® LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix® VC 713 (Hersteller: ISP), Gafquat®ASCP 1011, Gafquat®HS 110, Luviquat®8155 und Luviquat® MS 370 erhältlich sind.
Weitere in den erfindungsgemäßen Mitteln einsetzbare kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen® CMF, Hydagen® HCMF, Kytamer® PC und Chitolam® NB/101 im Handel verfügbar sind.
Erfindungsgemäß bevorzugte kationische Polymere sind kationische Cellulose-Derivate und Chitosan und dessen Derivate, insbesondere die Handelsprodukte Polymer®JR 400, Hydagen® HCMF und Kytamer® PC, kationische Guar-Derivate, kationische Honig-Derivate, insbesondere das Handelsprodukt Honeyquat® 50, kationische Alkylpolyglycodside gemäß der DE-PS 44 13 686 und Polymere vom Typ Polyquaternium-37.
Weiterhin sind kationisierte Proteinhydrolysate zu den kationischen Polymeren zu zählen, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann.
Ganz besonders bevorzugt sind die kationischen Proteinhydrolysate und -derivate auf pflanzlicher Basis.
Zusätzlich zu kationischen Polymerisaten oder an ihrer Stelle können die erfindungsgemäßen Mittel auch amphotere Polymere enthalten. Diese weisen zusätzlich mindestens eine negativ geladene Gruppe im Molekül auf und werden auch als zwitterionische Polymere bezeichnet. Im Rahmen der vorliegenden Erfindung bevorzugt einsetzbare zwitterionische Polymerisate setzen sich im wesentlichen zusammen aus
A) Monomeren mit quartären Ammoniumgruppen der allgemeinen Formel (Z-I),

   R¹-CH=CR²-CO-Z-(CₙH₂ₙ)-N⁽⁺⁾R³R⁴R⁵ A⁽⁻⁾ (Z-I)

   In der R¹ und R² unabhängig voneinander stehen für Wasserstoff oder eine Methylgruppe und R³, R⁴ und R⁵ unabhängig voneinander für Alkylgruppen mit 1 bis 4 Kohlenstoff-Atomen, Z eine NH-Gruppe oder ein Sauerstoffatom, n eine ganze Zahl von 2 bis 5 und A⁽⁻⁾ das Anion einer organischen oder anorganischen Säure ist und
B) monomeren Carbonsäuren der allgemeinen Formel (Z-II),

   R⁶-CH=CR⁷-COOH (II)

   in denen R⁶ und R⁷ unabhängig voneinander Wasserstoff oder Methylgruppen sind.
Geeignete Ausgangsmonomere sind z. B. Dimethylaminoethylacrylamid, Dimethylaminoethylmethacrylamid, Dimethylaminopropylacrylamid, Dimethylaminopropylmethacrylamid und Diethylaminoethylacrylamid, wenn Z eine NH-Gruppe bedeutet oder Dimethylaminoethylacrylat, Dimethylaminoethylmethacrylat und Diethylaminoethylacrylat, wenn Z ein Sauerstoffatom ist. Die eine tertiäre Aminogruppe enthaltenden Monomeren werden dann in bekannter Weise quaterniert, wobei als Alkylierungsreagenzien Methylchlorid, Dimethylsulfat oder Diethylsulfat besonders geeignet sind. Die Quaternisierungsreaktion kann in wässriger Lösung oder im Lösungsmittel erfolgen.
Vorteilhafterweise werden solche Monomere der Formel (Z-I) verwendet, die Derivate des Acrylamids oder Methacrylamids darstellen. Weiterhin bevorzugt sind solche Monomeren, die als Gegenionen Halogenid-, Methoxysulfat- oder Ethoxysulfat-Ionen enthalten. Ebenfalls bevorzugt sind solche Monomeren der Formel (Z-I), bei denen R³, R⁴ und R⁵ Methylgruppen sind. Das Acrylamidopropyltrimethylammoniumchlorid ist ein ganz besonders bevorzugtes Monomer der Formel (Z-I). Als monomere Carbonsäuren der Formel (Z-II) eignen sich Acrylsäure, Methacrylsäure, Crotonsäure und 2-Methyl-crotonsäure. Bevorzugt werden Acryl- oder Methacrylsäure, insbesondere Acrylsäure, eingesetzt.
Die kationischen bzw. amphoteren Polymere sind in den erfindungsgemäßen Mitteln bevorzugt in Mengen von 0,05 bis 10 Gew.-%, bezogen auf das gesamte Mittel, enthalten. Mengen von 0,1 bis 5 Gew.-% sind besonders bevorzugt.
Bei den anionischen Polymeren (G2) handelt es sich um anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure. Als ganz besonders wirkungsvoll haben sich anionische Polymere erwiesen, die als alleiniges oder Co-Monomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann. Besonders bevorzugt sind Homopolymere der 2-Acrylamido-2-methylpropansulfonsäure oder ihrer Salze, wie sie z. B. als Simulgel® 800 oder Viscolam AT 100 P erhältlich sind.
Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Dimethylacrylamid Methacrylamid, Acrylsäureester, Methacrylsäureester, Vinylpyrrolidon, Vinylether und Vinylester.
Bevorzugte erfindungsgemäße Zusammensetzungen enthalten mindestens ein anionisches verdickendes Polymer in Form eines inversen, auto-inversiblen Polymerlatex, enthaltend eine Ölphase, eine Wasserphase, mindestens einen Öl-in-Wasser-Emulgator und mindestens einen verzweigten oder vernetzten Polyelektrolyten, der ein Copolymer darstellt, das mindestens ein Monomer mit einer starken Säurefunktion sowie mindestens ein weiteres Monomer aufweist, das neutral ist oder eine schwache Säurefunktion enthält.
Bevorzugte Polyelektrolyten sind aus zwei, drei, vier, fünf oder sechs verschiedenen Monomeren aufgebaut, von denen mindestens ein Monomer eine starke Säurefunktion aufweist und das mindestens eine weitere Monomer neutral ist oder eine schwache Säurefunktion aufweist.
Bevorzugt sind die Monomerkombinationen
starke Säurefunktion/neutral,
starke Säurefunktion/schwache Säurefunktion,
starke Säurefunktion/schwache Säurefunktion/neutral
starke Säurefunktion/schwache Säurefunktion 1/schwache Säurefunktion 2/neutral,
starke Säurefunktion/schwache Säurefunktion/neutral 1/neutral 2,
starke Säurefunktion/schwache Säurefunktion 1/schwache Säurefunktion 2/neutral 1/neutral 2.
Die oben angegebene Reihenfolge der Auflistung der Monomerbausteine gibt dabei nicht notwendigerweise die tatsächliche Reihenfolge der Monomerbausteine im Polyelektrolytmolekül wieder.
In einer bevorzugten Ausführungsform der Erfindung sind die Polyelektrolytmonomeren, die eine schwache Säurefunktion enthalten, ausgewählt aus den Monomeren, die eine Carboxylgruppe -COOH enthalten, die teilweise oder vollständig neutralisiert ist.
Aus der partiellen Neutralisation der schwachen Säurefunktion resultieren der neutralisierte Monomerbaustein und der nicht-neutralisierte, saure Monomerbaustein, beispielsweise Acrylsäure und Natriumacrylat. Beide Monomere werden im Sinne der vorliegenden Anmeldung als gleich angesehen. Dementsprechend werden beide Monomere im Sinne der vorliegenden Anmeldung als "Monomer mit schwacher Säurefunktion" angesehen.
Besonders bevorzugte Beispiele für solche schwach sauren Polyelektrolytmonomeren sind Acrylsäure, Methacrylsäure, Itaconsäure und Maleinsäure. Bevorzugte erfindungsgemäße Zusammensetzungen sind daher dadurch gekennzeichnet, dass die schwache Säurefunktion des Polyelektrolytmonomeren eine Carboxylgruppe -COOH darstellt, die teilweise oder vollständig neutralisiert ist, wobei weiter bevorzugte erfindungsgemäße Zusammensetzungen dadurch gekennzeichnet sind, dass die Monomere des Polyelektrolyten, die mit der schwach sauren -COOH-Gruppe funktionalisiert sind, ausgewählt sind aus Acrylsäure, Methacrylsäure, Itaconsäure und Maleinsäure, die teilweise oder vollständig neutralisiert sind.
In einer weiteren bevorzugten Ausführungsform der vorliegenden Erfindung sind die Polyelektrolytmonomere mit einer starken Säuregruppe ausgewählt aus Monomeren, die mit einer Sulfonsäuregruppe -SO₃H oder einer Phosphonsäuregruppe funktionalisiert sind. Ein besonders bevorzugtes Polyelektrolytmonomer mit starker Säurefunktion ist 2-Methyl-2[(1-oxo-2-propenyl)amino]-1-propansulfonsäure, die teilweise oder vollständig neutralisiert ist.
Aus der partiellen Neutralisation der starken Säurefunktion resultieren der neutralisierte Monomerbaustein und der nicht-neutralisierte, saure Monomerbaustein, beispielsweise 2-Methyl-2[(1-oxo-2-propenyl)amino]-1-propansulfonsäure und Natrium-2-methyl-2[(1-oxo-2-propenyl)amino]-1-propansulfonat. Beide Monomere werden im Sinne der vorliegenden Anmeldung als gleich angesehen. Dementsprechend werden beide Monomere im Sinne der vorliegenden Anmeldung als "Monomer mit starker Säurefunktion" angesehen.
Bevorzugte erfindungsgemäße Zusammensetzungen sind daher dadurch gekennzeichnet, dass die starke Säurefunktion des Polyelektrolytmonomeren ausgewählt ist aus einer Sulfonsäuregruppe - SO₃H und einer Phosphonsäuregruppe, die teilweise oder vollständig neutralisiert sind, wobei weiter bevorzugte erfindungsgemäße Zusammensetzungen dadurch gekennzeichnet sind, dass das Polyelektrolytmonomer mit starker Säurefunktion ausgewählt ist aus 2-Methyl-2[(1-oxo-2-propenyl)amino]-1-propansulfonsäure, die teilweise oder vollständig neutralisiert ist.
Im Sinne der vorliegenden Erfindung sind alle Polyelektrolytmonomere, die mit einer schwachen oder starken Säuregruppe funktionalisiert sind, teilweise oder vollständig neutralisiert als Natrium-, Kalium-, Ammonium-, Ethanolamin- oder Aminosäure-Salz. Bevorzugte erfindungsgemäße Zusammensetzungen sind daher dadurch gekennzeichnet, dass die Polyelektrolytmonomere, die mit einer schwachen oder starken Säuregruppe funktionalisiert sind, teilweise oder vollständig neutralisiert sind als Natrium-, Kalium-, Ammonium-, Ethanolamin- oder Aminosäure-Salz.
In einer weiteren bevorzugten Ausführungsform der vorliegenden Erfindung ist das neutrale Polyelektrolytmonomer ausgewählt aus 2-Hydroxyethylacrylat, 2,3-Dihydroxypropylacrylat, 2-Hydroxyethylmethacrylat, 2,3-Dihydroxypropylmethacrylat, den ethoxylierten Derivaten der vorstehend genannten Ester mit einem Molekulargewicht zwischen 400 und 1000 g/mol, Acrylamid, Alkylacrylamiden, wie beispielsweise Methylacrylamid, Ethylacrylamid, n-Propylacrylamid und Isopropylacrylamid, Dialkylamiden, wie beispielsweise Dimethylacrylamid, Diethylacrylamid, Di-n-propylacrylamid und Diisopropylacrylamid, sowie Polyvinylpyrrolidon. In bevorzugten erfindungsgemäßen Zusammensetzungen ist das neutrale Polyelektrolytmonomer ausgewählt aus 2-Hydroxyethylacrylat, 2,3-Dihydroxypropylacrylat, 2-Hydroxyethylmethacrylat, 2,3-Dihydroxypropylmethacrylat, den ethoxylierten Derivaten der genannten Ester mit einem Molekulargewicht zwischen 400 und 1000 g/mol, Acrylamid, Dimethylacrylamid, Diethylacrylamid, Di-n-propylacrylamid, Diisopropylacrylamid sowie Polyvinylpyrrolidon. Besonders bevorzugte neutrale Polyelektrolytmonomere sind ausgewählt aus 2-Hydroxyethylacrylat, Acrylamid, Dimethylacrylamid und Polyvinylpyrrolidon. Vernetzte Copolymere aus 2-Methyl-2[(1-oxo-2-propenyl)amino]-1-propansulfonsäure (AMPS) und Hydroxyethylacrylat, in Form eines inversen, auto-inversiblen Latex sind kommerziell erhältlich, z. B. unter den Namen Simulgel®NS, Simulgel®I-NS 100, Simulgel®FL und Sepiplus® S von der Firma Seppic.

Weiterhin besonders bevorzugt sind teilweise oder vollständig neutralisierte, vernetzte Copolymere aus 2-Methyl-2[(1-oxo-2-propenyl)amino]-1-propansulfonsäure (AMPS) und Acrylamid, in Form eines inversen, auto-inversiblen Latex kommerziell erhältlich, z. B. unter dem Handelsnamen Simulgel®600 von der Firma Seppic. Weiterhin besonders bevorzugt sind teilweise oder vollständig neutralisierte, vernetzte Copolymere aus 2-Methyl-2[(1-oxo-2-propenyl)amino]-1-propansulfonsäure (AMPS) und Acrylsäure, in Form eines inversen, auto-inversiblen Latex erhältlich, z. B. unter den Namen Simulgel®EG und Simulgel®EPG von Seppic. Weiterhin besonders bevorzugt sind teilweise oder vollständig neutralisierte, vernetzte Copolymere aus 2-Methyl-2[(1-oxo-2-propenyl)amino]-1-propansulfonsäure (AMPS), Acrylsäure bzw. Natriumacrylat, und (als drittem Monomerbaustein) Dimethylacrylamid, in Form eines inversen, autoinversiblen Latex erhältlich, z. B. unter dem Namen Simulgel®SMS 88 von Seppic.

Die letztgenannten Polymere werden vorzugsweise in Mengen von von 0,05 bis 10 Gew.-%, vorzugsweise von 0,1 bis 5 Gew.-%, weiter bevorzugt von 0,15 bis 4 Gew.-%, noch weiter bevorzugt von 0,2 bis 3 Gew.-%, besonders bevorzugt von 0,25 bis 2 Gew.-% und insbesondere von 0,3 bis 1,5 Gew.-%, jeweils bezogen auf das gesamte Mittel, eingesetzt.

Allen vorgenannten, bevorzugt verwendeten inversen Polymerlatices ist gemein, dass sie mindestens ein Öl, bevorzugt ausgewählt aus weißen Mineralölen, Squalan, Isohexadecan und (gegebenenfalls hydriertem) Polyisobuten, sowie mindestens einen Öl-in-Wasser-Emulgator, ausgewählt aus den Ethylenoxid-Addukten von Sorbitanoleat, Ricinusöl, das gewünschtenfalls gehärtet ist, Sorbitanlaurat und Laurylalkohol, sowie weiterhin ausgewählt aus der Polyethylenoxid-freien Öl-in-Wasser-Emulgatorklasse der C6-C22-Fettalkohol-Glucoseether, insbesondere Caprinic Glucoside, Caprylic Glucoside, Capric Glucoside, Lauric Glucoside, Myristic Glucoside, Cetyl Glucoside, Stearyl Glucoside, Arachidyl Glucoside, Behenyl Glucoside, besonders bevorzugt Caprylic Glucoside und Capric Glucoside, enthalten. Demnach sind bevorzugte erfindungsgemäße Zusammensetzungen dadurch gekennzeichnet, dass die Ölphase des inversen Polymerlatex mindestens ein Öl, ausgewählt aus weißen Mineralölen, Squalan, Isohexadecan und (gegebenenfalls hydriertem) Polyisobuten, enthält. Weiter bevorzugte erfindungsgemäße Zusammensetzungen sind dadurch gekennzeichnet, dass der in dem inversen Polymerlatex enthaltene Öl-in-Wasser-Emulgator ausgewählt ist aus den Ethylenoxid-Addukten von Sorbitanoleat, Ricinusöl, das gewünschtenfalls gehärtet ist, Sorbitanlaurat und Laurylalkohol, sowie aus Caprylic Glucoside und Capric Glucoside. Die erfindungsgemäß bevorzugten verdickenden Polymerlatices weisen vorzugsweise einen Polymergehalt von 30 - 90 Gew.-%, bevorzugt 35 - 75 Gew.-% und besonders bevorzugt 40 - 60 Gew.-%, jeweils bezogen auf den gesamten Latex, auf. Der Polymergehalt des Latex hat dabei jedoch eher nur eine Bedeutung für die Herstellung der erfindungsgemäßen Zusammensetzungen, z. B. in Bezug auf die Mischbarkeit bzw. Dosierbarkeit. Für die erfindungsgemäßen Zusammensetzungen selbst ist eher der Polymergehalt an sich, bezogen auf die erfindungsgemäße Zusammensetzung, bedeutsam.
Bezogen auf die gesamte erfindungsgemäße kosmetische Zusammensetzung, wird das verdickende Polymer in Mengen von 0,1 - 5 Gew.-%, bevorzugt 0,3 - 3 Gew.-% und besonders bevorzugt 0,5 - 2,5 Gew.-%, bezogen auf die gesamte erfindungsgemäße kosmetische Zusammensetzung, eingesetzt, wobei Polymergehalte von 0,4, 0,6, 0,7, 0,8, 0,9, 1,0, 1,1, 1,2, 1,3, 1,4, 1,5, 1,6, 1,7, 1,8, 1,9, 2,0, 2,1, 2,2, 2,3 und 2,4 Gew.-% besonders bevorzugt und Polymergehalte von 0,3, 0,4, 0,5, 0,6, 0,7, 0,8, 0,9, 1,0 und 1,1 Gew.-% außerordentlich bevorzugt sein können.
Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol® im Handel erhältlich.
Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnung Stabileze® QM im Handel erhältlich.
Weiterhin können als Polymere zur Steigerung der Wirkung der erfindungsgemäßen Wirkstoffkombination amphotere Polymere (G3) verwendet werden. Unter dem Begriff amphotere Polymere werden sowohl solche Polymere, die im Molekül sowohl freie Aminogruppen als auch freie -COOH- oder SO₃H-Gruppen enthalten und zur Ausbildung innerer Salze befähigt sind, als auch zwitterionische Polymere, die im Molekül quartäre Ammoniumgruppen und -COO⁻- oder -SO₃⁻-Gruppen enthalten, und solche Polymere zusammengefaßt, die -COOH- oder SO₃H-Gruppen und quartäre Ammoniumgruppen enthalten.
Ein Beispiel für ein erfindungsgemäß einsetzbares Amphopolymer ist das unter der Bezeichnung Amphomer® erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.
Bevorzugt eingesetzte amphotere Polymere sind solche Polymerisate, die sich im wesentlichen zusammensetzen aus
(a) Monomeren mit quartären Ammoniumgruppen der allgemeinen Formel (G3-I),

   R¹-CH=CR²-CO-Z-(CₙH₂ₙ)-N⁽⁺⁾R³R⁴R⁵A⁽⁻⁾ (G3-I)

   in der R¹ und R² unabhängig voneinander stehen für Wasserstoff oder eine Methylgruppe und R³, R⁴ und R⁵ unabhängig voneinander für Alkylgruppen mit 1 bis 4 Kohlenstoffatomen, Z eine NH-Gruppe oder ein Sauerstoffatom, n eine ganze Zahl von 2 bis 5 und A⁽⁻⁾ das Anion einer organischen oder anorganischen Säure ist, und
(b) monomeren Carbonsäuren der allgemeinen Formel (G3-II),

   R⁶-CH=CR⁷-COOH (G3-II)

   in denen R⁶ und R⁷ unabhängig voneinander Wasserstoff oder Methylgruppen sind. Diese Verbindungen können sowohl direkt als auch in Salzform, die durch Neutralisation der Polymerisate, beispielsweise mit einem Alkalihydroxid, erhalten wird, erfindungsgemäß eingesetzt werden. Ganz besonders bevorzugt sind solche Polymerisate, bei denen Monomere des Typs (a) eingesetzt werden, bei denen R³, R⁴ und R⁵ Methylgruppen sind, Z eine NH-Gruppe und A⁽⁻⁾ ein Halogenid-, Methoxysulfat- oder Ethoxysulfat-Ion ist; Acrylamidopropyl-trimethyl-ammoniumchlorid ist ein besonders bevorzugtes Monomeres (a). Als Monomeres (b) für die genannten Polymerisate wird bevorzugt Acrylsäure verwendet.
Die erfindungsgemäßen Mittel können in einer weiteren bevorzugten Ausführungsform nichtionogene Polymere (G4) enthalten. Geeignete nichtionogene Polymere sind beispielsweise:
- Vinylpyrrolidon/Vinylester-Copolymere, wie sie beispielsweise unter dem Warenzeichen Luviskol® (BASF) vertrieben werden. Luviskol® VA 64 und Luviskol® VA 73, jeweils Vinylpyrrolidon/Vinylacetat-Copolymere, sind ebenfalls bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal® und Benecel® (AQUALON) und Natrosol®-Typen (Hercules) vertrieben werden.
- Stärke und deren Derivate, insbesondere Stärkealkylether;
- Polyvinylpyrrolidone, wie sie beispielsweise unter der Bezeichnung Luviskol® (BASF) vertrieben werden.
Es ist erfindungsgemäß auch möglich, dass die verwendeten Zusammensetzungen mehrere, insbesondere zwei verschiedene Polymere gleicher Ladung und/oder jeweils ein ionisches und ein amphoteres und/oder nicht ionisches Polymer enthalten.
Die Polymere (G) sind in den erfindungsgemäßen Mitteln bevorzugt in Mengen von 0,05 bis 10 Gew.-%, bezogen auf das gesamte Mittel, enthalten. Mengen von 0,1 bis 5, insbesondere von 0,1 bis 3 Gew.-%, sind besonders bevorzugt.
Eine weitere Gruppe von Pflegestoffen, die in den erfindungsgemäßen Mitteln enthalten sein kann, sind die Proteinhydrolysate und deren Derivate (P) enthalten. Proteinhydrolysate sind Produktgemische, die durch sauer, basisch oder enzymatisch katalysierten Abbau von Proteinen erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid. Selbstverständlich können erfindungsgemäß auch β-Aminosäuren und deren Derivate wie β-Alanin, Anthranilsäure oder Hippursäure eingesetzt werden. Das Molgewicht der erfindungsgemäß einsetzbaren Proteinhydrolysate liegt zwischen 75, dem Molgewicht für Glycin, und 200000, bevorzugt beträgt das Molgewicht 75 bis 50000 und ganz besonders bevorzugt 75 bis 20000 Dalton.
Erfindungsgemäß können Proteinhydrolysate sowohl pflanzlichen als auch tierischen oder marinen oder synthetischen Ursprungs eingesetzt werden. Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte. Selbstverständlich umfasst die erfindungsgemäße Lehre alle isomeren Formen, wie cis - trans - Isomere, Diastereomere und chirale Isomere. Erfindungsgemäß ist es auch bevorzugt, eine Mischung aus mehreren Proteinhydrolysaten (P) einzusetzen. Die Proteinhydrolysate (P) sind in den Mitteln bevorzugt in Konzentrationen von 0,01 Gew.-% bis zu 20 Gew.-%, besonders bevorzugt von 0,05 Gew.- bis zu 15 Gew.-% und ganz besonders bevorzugt in Mengen von 0,05 Gew.-% bis zu 5 Gew.-% enthalten.
Die erfindungsgemäßen Mittel können weiterhin bevorzugt eine 2-Pyrrolidinon-5-carbonsäure und deren Derivate (J) enthalten. Besonders bevorzugt sind die Natrium-, Kalium-, Calcium-, Magnesium- oder Ammoniumsalze, bei denen das Ammoniumion neben Wasserstoff eine bis drei C₁- bis C₄-Alkylgruppen trägt. Das Natriumsalz ist ganz besonders bevorzugt. Die eingesetzten Mengen in den erfindungsgemäßen Mitteln betragen vorzugsweise 0,05 bis 10 Gew.-%, bezogen auf das gesamte Mittel, besonders bevorzugt 0,1 bis 5, und insbesondere 0,1 bis 3 Gew.-%.
Zusätzlich kann es sich als vorteilhaft erweisen, wenn in den erfindungsgemäßen Zusammensetzungen mindestens ein wasserlösliches mehrwertiges C₂ - C₉-Alkanol mit 2 - 6 Hydroxylgruppen und/oder mindestens ein wasserlösliches Polyethylenglycol mit 3 - 20 Ethylenoxid-Einheiten sowie Mischungen hiervon enthalten ist. Derartige Polyole können ein besonders ebenmäßiges Ergebnis der Hautaufhellung begünstigen.
Erfindungsgemäß wird unter Wasserlöslichkeit eine Löslichkeit von wenigstens 5 Gew.-% bei 20 °C verstanden, das heißt, dass Mengen von wenigstens 5 g des mehrwertigen C₂ - C₉-Alkanols mit 2 - 6 Hydroxylgruppen bzw. des Polyethylenglycols mit 3 - 20 Ethylenoxid-Einheiten in 95 g Wasser bei 20 °C löslich sind.
Bevorzugt sind diese Komponenten ausgewählt aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,2-Hexandiol und 1,6-Hexandiol, Hexantriolen wie 1,2,6-Hexantriol, 2-Ethyl-2-hydroxymethyl-1,3-propandiol, 1,2-Octandiol, 1,8-Octandiol, Diethylenglycol, Triethylenglycol, Dipropylenglycol, Tripropylenglycol, Diglycerin, Triglycerin, Erythrit, Sorbit sowie Mischungen der vorgenannten Substanzen.

Erfindungsgemäß besonders wirksame und damit bevorzugt kosmetische Mittel sind dadurch gekennzeichnet, daß sie, bezogen auf das Gewicht des anwendungsbereiten Mittels 0,001 bis 20 Gew.-%, vorzugsweise 0,01 bis 17,5 Gew.-%, weiter bevorzugt 0,1 bis 15 Gew.-%, noch weiter bevorzugt 0,5 bis 10 Gew.-% und insbesondere 1 bis 5 Gew.-% einer oder mehrerer Verbindungt(en) aus der Gruppe 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,2-Hexandiol und 1,6-Hexandiol, Hexantriolen wie 1,2,6-Hexantriol, 2-Ethyl-2-hydroxymethyl-1,3-propandiol, 1,2-Octandiol, 1,8-Octandiol, Diethylenglycol, Triethylenglycol, Dipropylenglycol, Tripropylenglycol, Diglycerin, Triglycerin, Erythrit, Sorbit sowie Mischungen der vorgenannten Substanzen enthalten.

Geeignete wasserlösliche Polyethylenglycole sind ausgewählt aus PEG-3, PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 bevorzugt sind. Auch Zucker und bestimmte Zuckerderivate wie Fructose, Glucose, Maltose, Maltitol, Mannit, Inosit, Sucrose, Trehalose und Xylose sind erfindungsgemäß geeignet.

Erfindungsgemäß besonders wirksame und damit bevorzugt kosmetische Mittel sind dadurch gekennzeichnet, daß sie, bezogen auf das Gewicht des anwendungsbereiten Mittels 0,001 bis 20 Gew.-%, vorzugsweise 0,01 bis 17,5 Gew.-%, weiter bevorzugt 0,1 bis 15 Gew.-%, noch weiter bevorzugt 0,5 bis 10 Gew.-% und insbesondere 1 bis 5 Gew.-% Polyethylenglycol(e) aus der Gruppe PEG-3, PEG-4, PEG-6, PEG-7, PEG-8 sowie Mischungen hiervon enthalten.

Die erfindungsgemäßen Zusammensetzungen können Parfüme, Parfümöle oder Parfümölbestandteile enthalten.

Die erfindungsgemäßen Zusammensetzungen eignen sich zur Aufhellung der Haut und können dementsprechend eingesetzt werden. Dabei kann die Haut lokal begrenzt (z.B. auf Sommersprossen, Altersflecken oder Fehlpigmentierungen) oder großflächig (zur Hautaufhellung) behandelt werden.

Weitere Gegenstände der vorliegenden Erfindung sind daher die Verwendung von erfindungsgemäßen kosmetischen oder dermatologischen Zusammensetzungen zur Aufhellung der Hautfarbe, zur Reduzierung der Pigmentierung der Haut, zum Ausgleich des Erscheinungsbildes einer ungleichmäßigen Hautpigmentierung und/oder zur Aufhellung von Altersflecken der Haut.

Ein weiterer Gegenstand der vorliegenden Erfindung ist die Verwendung von erfindungsgemäßen kosmetischen und dermatologischen Zusammensetzungen zur Behandlung postinflammatorischer Hyperpigmentierung.

Bezüglich bevorzugter Ausführungsformen der erfindungsgemäßen Verwendungen gilt mutatis mutandis das zu den erfindungsgemäßen Zusammensetzungen Gesagte.

Ein weiterer Gegenstand der vorliegenden Erfindung ist ein nicht-therapeutisches, kosmetisches Verfahren zur Aufhellung der Haut und/oder zur Aufhellung von Altersflecken oder Sommersprossen, dadurch gekennzeichnet, daß ein kosmetisches Mittel, enthaltend
a) mindestens einen Extrakt aus Opuntia Ficus Indica;
b) mindestens einen Extrakt aus Brassicae-Pflanzen;
c) 2,3-Dihydro-5,6-dimethoxy-3,3-dimethyl-2-(phenylmethylen)-1H-Inden-1-on (Benzyliden Dimethoxydimethylindanon) auf die Haut aufgetragen wird.

Bezüglich bevorzugter Ausführungsformen des erfindungsgemäßen Verfahrens gilt mutatis mutandis das zu den erfindungsgemäßen Zusammensetzungen Gesagte.

## Patentansprüche

1. Kosmetisches Mittel zur Aufhellung der Haut und/oder von Altersflecken, enthaltend
a) mindestens einen Extrakt aus Opuntia Ficus Indica;
b) mindestens einen Extrakt aus Brassicae-Pflanzen;
c) 2,3-Dihydro-5,6-dimethoxy-3,3-dimethyl-2-(phenylmethylen)-1H-Inden-1-on (Benzyliden Dimethoxydimethylindanon).

2. Kosmetisches Mittel nach Anspruch 1, **dadurch gekennzeichnet, daß** es, bezogen auf das Gewicht des anwendungsbereiten Mittels 0,001 bis 20 Gew.-%, vorzugsweise 0,005 bis 15 Gew.-%, weiter bevorzugt 0,01 bis 10 Gew.-%, noch weiter bevorzugt 0,05 bis 5 Gew.-% und insbesondere 0,1 bis 1 Gew.-% mindestens eines Extraktes aus Opuntia Ficus Indica enthält.

3. Kosmetisches Mittel nach einem der Ansprüche 1 oder 2, **dadurch gekennzeichnet, daß** es, bezogen auf das Gewicht des anwendungsbereiten Mittels 0,001 bis 20 Gew.-%, vorzugsweise 0,005 bis 15 Gew.-%, weiter bevorzugt 0,01 bis 10 Gew.-%, noch weiter bevorzugt 0,05 bis 5 Gew.-% und insbesondere 0,1 bis 1 Gew.-% mindestens eines ganz oder teilweise hydrolysierten Extraktes aus Opuntia Ficus Indica (CAS-Nr. 90082-21-6) enthält.

4. Kosmetisches Mittel nach einem der Ansprüche 1 bis 3, **dadurch gekennzeichnet, daß** es, bezogen auf das Gewicht des anwendungsbereiten Mittels 0,001 bis 20 Gew.-%, vorzugsweise 0,01 bis 17,5 Gew.-%, weiter bevorzugt 0,1 bis 15 Gew.-%, noch weiter bevorzugt 0,5 bis 10 Gew.-% und insbesondere 1 bis 5 Gew.-% mindestens eines Extraktes aus Brassicae-Pflanzen enthält.

5. Kosmetisches Mittel nach einem der Ansprüche 1 bis 4, **dadurch gekennzeichnet, daß** es, bezogen auf das Gewicht des anwendungsbereiten Mittels 0,001 bis 20 Gew.-%, vorzugsweise 0,01 bis 17,5 Gew.-%, weiter bevorzugt 0,1 bis 15 Gew.-%, noch weiter bevorzugt 0,5 bis 10 Gew.-% und insbesondere 1 bis 5 Gew.-% mindestens eines Extraktes aus Brassica Napus enthält.

6. Kosmetisches Mittel nach einem der Ansprüche 1 bis 5, **dadurch gekennzeichnet, daß** es, bezogen auf das Gewicht des anwendungsbereiten Mittels 0,001 bis 20 Gew.-%, vorzugsweise 0,01 bis 17,5 Gew.-%, weiter bevorzugt 0,1 bis 15 Gew.-%, noch weiter bevorzugt 0,5 bis 10 Gew.-% und insbesondere 1 bis 5 Gew.-% mindestens eines ganz oder teilweise hydrolysierten Extraktes aus fermentierten Pflanzenteilen von Brassica Napus enthält.

7. Kosmetisches Mittel nach einem der Ansprüche 1 bis 6, **dadurch gekennzeichnet, daß** es, bezogen auf das Gewicht des anwendungsbereiten Mittels 0,001 bis 20 Gew.-%, vorzugsweise 0,01 bis 17,5 Gew.-%, weiter bevorzugt 0,1 bis 15 Gew.-%, noch weiter bevorzugt 0,5 bis 10 Gew.-% und insbesondere 1 bis 5 Gew.-% 2,3-Dihydro-5,6-dimethoxy-3,3-dimethyl-2-(phenylmethylen)-1H-Inden-1-on (Benzyliden Dimethoxydimethylindanon) enthält.

8. Kosmetisches Mittel nach einem der Ansprüche 1 bis 7, **dadurch gekennzeichnet, daß** das Gewichtsverhältnis von b) zu a) > 1 zu 1, vorzugsweise > 1,1 zu 1, weiter bevorzugt > 1,25 zu 1 und insbesondere > 1,5 zu 1 beträgt.

9. Kosmetisches Mittel nach einem der Ansprüche 1 bis 8, **dadurch gekennzeichnet, daß** es, bezogen auf das Gewicht des anwendungsbereiten Mittels 10 bis 70 Gew.-%, vorzugsweise 20 bis 65 Gew.-%, weiter bevorzugt 30 bis 60 Gew.-%, noch weiter bevorzugt 35 bis 55 Gew.-% und insbesondere 40 bis 50 Gew.-% Wasser enthält.

10. Kosmetisches Mittel nach einem der Ansprüche 1 bis 9, **dadurch gekennzeichnet, daß** es, bezogen auf das Gewicht des anwendungsbereiten Mittels 0,001 bis 20 Gew.-%, vorzugsweise 0,01 bis 17,5 Gew.-%, weiter bevorzugt 0,1 bis 15 Gew.-%, noch weiter bevorzugt 0,5 bis 10 Gew.-% und insbesondere 1 bis 5 Gew.-% einer oder mehrerer Verbindungt(en) aus der Gruppe 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,2-Hexandiol und 1,6-Hexandiol, Hexantriolen wie 1,2,6-Hexantriol, 2-Ethyl-2-hydroxymethyl-1,3-propandiol, 1,2-Octandiol, 1,8-Octandiol, Diethylenglycol, Triethylenglycol, Dipropylenglycol, Tripropylenglycol, Diglycerin, Triglycerin, Erythrit, Sorbit sowie Mischungen der vorgenannten Substanzen enthält.

11. Kosmetisches Mittel nach Anspruch 10, **dadurch gekennzeichnet, daß** es, bezogen auf das Gewicht des anwendungsbereiten Mittels 0,001 bis 20 Gew.-%, vorzugsweise 0,01 bis 17,5 Gew.-%, weiter bevorzugt 0,1 bis 15 Gew.-%, noch weiter bevorzugt 0,5 bis 10 Gew.-% und insbesondere 1 bis 5 Gew.-% Polyethylenglycol(e) aus der Gruppe PEG-3, PEG-4, PEG-6, PEG-7, PEG-8 sowie Mischungen hiervon enthält.

12. Kosmetisches Mittel nach einem der Ansprüche 1 bis 11, **dadurch gekennzeichnet, daß** es eine Viskosität (Brookfield-Viskosimeter LVT-II bei 20 U/min und 20°C, Spindel 3) von 50 bis 50000 mPa·s, vorzugsweise 1000 bis 30000 mPas und insbesondere von 5000 bis 20000 mPas aufweist.

13. Kosmetisches Mittel nach einem der Ansprüche 1 bis 12, **dadurch gekennzeichnet, daß** es als Emulsion vom Typ Wasser-in-ÖI (W/O), als Emulsion vom Typ Öl-in-Wasser (O/W), als multiple Emulsion, beispielsweise vom Typ Wasser-in-ÖI-in-Wasser (W/O/W) oder Öl-in-Wasser-in-Öl (O/W/O) konfektioniert ist.

14. Kosmetisches Mittel nach einem der Ansprüche 1 bis 13, **dadurch gekennzeichnet, daß** bezogen auf das Gewicht des anwendungsbereiten Mittels, 0,05 bis 10 Gew.-%, vorzugsweise 0,1 bis 5 Gew.-%, weiter bevorzugt 0,15 bis 4 Gew.-%, noch weiter bevorzugt 0,2 bis 3 Gew.-%, besonders bevorzugt 0,25 bis 2 Gew.-% und insbesondere 0,3 bis 1,5 Gew.-%, teilweise oder vollständig neutralisierte, vernetzte Copolymere aus 2-Methyl-2[(1-oxo-2-propenyl)amino]-1-propansulfonsäure (AMPS) und Acrylsäure enthält.

15. Nicht-therapeutisches, kosmetisches Verfahren zur Aufhellung der Haut und/oder zur Aufhellung von Altersflecken oder Sommersprossen, **dadurch gekennzeichnet, daß** ein kosmetisches Mittel, enthaltend
a) mindestens einen Extrakt aus Opuntia Ficus Indica;
b) mindestens einen Extrakt aus Brassicae-Pflanzen;
c) 2,3-Dihydro-5,6-dimethoxy-3,3-dimethyl-2-(phenylmethylen)-1H-Inden-1-on (Benzyliden Dimethoxydimethylindanon)
auf die Haut aufgetragen wird.

## Claims

1. A cosmetic agent for lightening the skin and/or age spots, containing
a) at least one extract from Opuntia Ficus Indica;
b) at least one extract from Brassica plants;
c) 2,3-dihydro-5,6-dimethoxy-3,3-dimethyl-2-(phenylmethylene)-1H-Inden-1-one (benzylidene dimethoxydimethylindanone).

2. The cosmetic agent according to claim 1, **characterized in that** it contains, based on the weight of the ready-to-apply agent, from 0.001 to 20 wt.%, preferably from 0.005 to 15 wt.%, more preferably from 0.01 to 10 wt.%, even more preferably from 0.05 to 5 wt.%, and in particular from 0.1 to 1 wt.%, of at least one extract from Opuntia Ficus Indica.

3. The cosmetic agent according to one of claims 1 or 2, **characterized in that** it contains, based on the weight of the ready-to-apply agent, from 0.001 to 20 wt.%, preferably from 0.005 to 15 wt.%, more preferably from 0.01 to 10 wt.%, even more preferably from 0.05 to 5 wt.%, and in particular from 0.1 to 1 wt.%, of at least one completely or partially hydrolyzed extract from Opuntia Ficus Indica (CAS no. 90082-21-6).

4. The cosmetic agent according to one of claims 1 to 3, **characterized in that** it contains, based on the weight of the ready-to-apply agent, from 0.001 to 20 wt.%, preferably from 0.01 to 17.5 wt.%, more preferably from 0.1 to 15 wt.%, even more preferably from 0.5 to 10 wt.%, and in particular from 1 to 5 wt.%, of at least one extract from Brassica plants.

5. The cosmetic agent according to one of claims 1 to 4, **characterized in that** it contains, based on the weight of the ready-to-apply agent, from 0.001 to 20 wt.%, preferably from 0.01 to 17.5 wt.%, more preferably from 0.1 to 15 wt.%, even more preferably from 0.5 to 10 wt.%, and in particular from 1 to 5 wt.%, of at least one extract from Brassica Napus.

6. The cosmetic agent according to one of claims 1 to 5, **characterized in that** it contains, based on the weight of the ready-to-apply agent, from 0.001 to 20 wt.%, preferably from 0.01 to 17.5 wt.%, more preferably from 0.1 to 15 wt.%, even more preferably from 0.5 to 10 wt.%, and in particular from 1 to 5 wt.%, of at least one completely or partially hydrolyzed extract from fermented plant parts of Brassica Napus.

7. The cosmetic agent according to one of claims 1 to 6, **characterized in that** it contains, based on the weight of the ready-to-apply agent, from 0.001 to 20 wt.%, preferably from 0.01 to 17.5 wt.%, more preferably from 0.1 to 15 wt.%, even more preferably from 0.5 to 10 wt.%, and in particular from 1 to 5 wt.%, of 2,3-dihydro-5,6-dimethoxy-3,3-dimethyl-2-(phenylmethylene)-1H-Inden-1-one (benzylidene dimethoxydimethylindanone).

8. The cosmetic agent according to one of claims 1 to 7, **characterized in that** the weight ratio of b) to a) is > 1 to 1, preferably > 1.1 to 1, more preferably > 1.25 to 1, and in particular > 1.5 to 1.

9. The cosmetic agent according to one of claims 1 to 8, **characterized in that** it contains, based on the weight of the ready-to-apply agent, from 10 to 70 wt.%, preferably from 20 to 65 wt.%, more preferably from 30 to 60 wt.%, even more preferably from 35 to 55 wt.%, and in particular from 40 to 50 wt.%, of water.

10. The cosmetic agent according to one of claims 1 to 9, **characterized in that** it contains, based on the weight of the ready-to-apply agent, from 0.001 to 20 wt.%, preferably from 0.01 to 17.5 wt.%, more preferably from 0.1 to 15 wt.%, even more preferably from 0.5 to 10 wt.%, and in particular from 1 to 5 wt.%, of one or more compound(s) from the group of 1,2-propylene glycol, 2-methyl-1,3-propanediol, glycerol, butylene glycols such as 1,2-butylene glycol, 1,3-butylene glycol and 1,4-butylene glycol, pentylene glycols such as 1,2-pentanediol and 1,5-pentanediol, hexanediols such as 1,2-hexanediol and 1,6-hexanediol, hexanetriols such as 1,2,6-hexanetriol, 2-ethyl-2-hydroxymethyl-1,3-propanediol, 1,2-octanediol, 1,8-octanediol, diethylene glycol, triethylene glycol, dipropylene glycol, tripropylene glycol, diglycerol, triglycerol, erythritol, sorbitol, and mixtures of the aforementioned substances.

11. The cosmetic agent according to claim 10, **characterized in that** it contains, based on the weight of the ready-to-apply agent, from 0.001 to 20 wt.%, preferably from 0.01 to 17.5 wt.%, more preferably from 0.1 to 15 wt.%, even more preferably from 0.5 to 10 wt.%, and in particular from 1 to 5 wt.%, of polyethylene glycol(s) from the group of PEG-3, PEG-4, PEG-6, PEG-7, PEG-8 and mixtures thereof.

12. The cosmetic agent according to one of claims 1 to 11, **characterized in that** it has a viscosity (Brookfield viscometer LVT-II at 20 rpm and 20 °C, spindle 3) of from 50 to 50,000 mPas, preferably from 1000 to 30,000 mPas, and in particular from 5000 to 20,000 mPas.

13. The cosmetic agent according to one of claims 1 to 12, **characterized in that** it is formulated as an emulsion of the water-in-oil (W/O) type, as an emulsion of the oil-in-water (O/W) type, or as a multiple emulsion, for example of the water-in-oil-in-water (W/O/W) type or oil-in-water-in-oil (O/W/O) type.

14. The cosmetic agent according to one of claims 1 to 13, **characterized in that** it contains, based on the weight of the ready-to-apply agent, from 0.05 to 10 wt.%, preferably from 0.1 to 5 wt.%, more preferably from 0.15 to 4 wt.%, even more preferably from 0.2 to 3 wt.%, particularly preferably from 0.25 to 2 wt.%, and in particular from 0.3 to 1.5 wt.%, of partially or completely neutralized, crosslinked copolymers from 2-methyl-2[(1-oxo-2-propenyl)amino]-1-propanesulfonic acid (AMPS) and acrylic acid.

15. A non-therapeutic, cosmetic method for lightening the skin and/or for lightening age spots or freckles, **characterized in that** a cosmetic agent containing
a) at least one extract from Opuntia Ficus Indica;
b) at least one extract from Brassica plants;
c) 2,3-dihydro-5,6-dimethoxy-3,3-dimethyl-2-(phenylmethylene)-1H-Inden-1-one (benzylidene dimethoxydimethylindanone)
is applied to the skin.

## Revendications

1. Agent cosmétique pour l'éclaircissement de la peau et/ou de lentigos séniles contenant
a) au moins un extrait d'Opuntia Ficus Indica ;
b) au moins un extrait de plantes de la famille des Brassicae ;
c) 2,3-dihydro-5,6-diméthoxy-3,3-diméthyl-2-(phénylméthyléno)-1H-indéno-1-one (benzylidène-diméthoxydiméthylindanone).

2. Agent cosmétique selon la revendication 1, **caractérisé en ce qu'**il contient, par rapport au poids de l'agent prêt à l'emploi, 0,001 à 20 % en poids, de préférence 0,005 à 15 % en poids, de manière davantage préférée 0,01 à 10 % en poids, de manière encore davantage préférée 0,05 à 5 % en poids et en particulier 0,1 à 1 % en poids d'au moins un extrait d'Opuntia Ficus Indica.

3. Agent cosmétique selon l'une des revendications 1 ou 2, **caractérisé en ce qu'**il contient, par rapport au poids de l'agent prêt à l'emploi, 0,001 à 20 % en poids, de préférence 0,005 à 15 % en poids, de manière davantage préférée 0,01 à 10 % en poids, de manière encore davantage préférée 0,05 à 5 % en poids et en particulier 0,1 à 1 % en poids d'au moins un extrait totalement ou partiellement hydrolysé d'Opuntia Ficus Indica (n° CAS 90082-21-6).

4. Agent cosmétique selon l'une des revendications 1 à 3, **caractérisé en ce qu'**il contient, par rapport au poids de l'agent prêt à l'emploi, 0,001 à 20 % en poids, de préférence 0,01 à 17,5 % en poids, de manière davantage préférée 0,1 à 15 % en poids, de manière encore davantage préférée 0,5 à 10 % en poids et en particulier 1 à 5 % en poids d'au moins un extrait de plantes de la famille des Brassicae.

5. Agent cosmétique selon l'une des revendications 1 à 4, **caractérisé en ce qu'**il contient, par rapport au poids de l'agent prêt à l'emploi, 0,001 à 20 % en poids, de préférence 0,01 à 17,5 % en poids, de manière davantage préférée 0,1 à 15 % en poids, de manière encore davantage préférée 0,5 à 10 % en poids et en particulier 1 à 5 % en poids d'au moins un extrait de Brassica Napus.

6. Agent cosmétique selon l'une des revendications 1 à 5, **caractérisé en ce qu'**il contient, par rapport au poids de l'agent prêt à l'emploi, 0,001 à 20 % en poids, de préférence 0,01 à 17,5 % en poids, de manière davantage préférée 0,1 à 15 % en poids, de manière encore davantage préférée 0,5 à 10 % en poids et en particulier 1 à 5 % en poids d'au moins un extrait totalement ou partiellement hydrolysé de parties de plantes fermentées de Brassica Napus.

7. Agent cosmétique selon l'une des revendications 1 à 6, **caractérisé en ce qu'**il contient, par rapport au poids de l'agent prêt à l'emploi, 0,001 à 20 % en poids, de préférence 0,01 à 17,5 % en poids, de manière davantage préférée 0,1 à 15 % en poids, de manière encore davantage préférée 0,5 à 10 % en poids et en particulier 1 à 5 % en poids de 2,3-dihydro-5,6-diméthoxy-3,3-diméthyl-2-(phénylméthyléno)-1H-indéno-1-one (benzylidène-diméthoxydiméthylindanone).

8. Agent cosmétique selon l'une des revendications 1 à 7, **caractérisé en ce que** le rapport pondéral entre b) et a) est > 1 sur 1, de préférence > 1,1 sur 1, de manière davantage préférée > 1,25 sur 1 et en particulier > 1,5 sur 1.

9. Agent cosmétique selon l'une des revendications 1 à 8, **caractérisé en ce qu'**il contient, par rapport au poids de l'agent prêt à l'emploi, 10 à 70 % en poids, de préférence 20 à 65 % en poids, de manière davantage préférée 30 à 60 % en poids, de manière encore davantage préférée 35 à 55 % en poids et en particulier 40 à 50 % en poids d'eau.

10. Agent cosmétique selon l'une des revendications 1 à 9, **caractérisé en ce qu'**il contient, par rapport au poids de l'agent prêt à l'emploi, 0,001 à 20 % en poids, de préférence 0,01 à 17,5 % en poids, de manière davantage préférée 0,1 à 15 % en poids, de manière encore davantage préférée 0,5 à 10 % en poids et en particulier 1 à 5 % en poids d'un ou plusieurs composé(s) du groupe comprenant le 1,2-propylène glycol, le 2-méthyl-1,3-propanediol, le glycérol, des butylènes glycols tels que le 1,2-butylène glycol, le 1,3-butylène glycol et le 1,4-butylène glycol, des pentylènes glycols tels que le 1,2-pentanediol et le 1,5-pentandiol, des hexanediols tels que le 1,2-hexanediol et le 1,6-hexanediol, des hexanetriols tels que le 1,2,6-hexanetriol, le 2-éthyl-2-hydroxyméthyl-1,3-propanediol, le 1,2-octanediol, le 1,8-octanediol, le diéthylèneglycol, le triéthylèneglycol, le dipropylèneglycol, le tripropylèneglycol, le diglycérol, le triglycérol, l'érythritol, le sorbitol ainsi que des mélanges des substances susmentionnées.

11. Agent cosmétique selon la revendication 10, **caractérisé en ce qu'**il contient, par rapport au poids de l'agent prêt à l'emploi, 0,001 à 20 % en poids, de préférence 0,01 à 17,5 % en poids, de manière davantage préférée 0,1 à 15 % en poids, de manière encore davantage préférée 0,5 à 10 % en poids et en particulier 1 à 5 % en poids de polyéthylèneglycol(s) issu(s) du groupe des PEG-3, PEG-4, PEG-6, PEG-7, PEG-8 ainsi que des mélanges de ces derniers.

12. Agent cosmétique selon l'une des revendications 1 à 11, **caractérisé en ce qu'**il présente une viscosité (viscosimètre Brookfield LVT-II à 20 tr/min et 20°C, broche 3) de 50 à 50 000 mPa·s, de préférence 1 000 à 30 000 mPa·s et en particulier de 5 000 à 20 000 mPa·s.

13. Agent cosmétique selon l'une des revendications 1 à 12, **caractérisé en ce qu'**il est préparé sous forme d'émulsion de type eau-dans-l'huile (E/H), sous forme d'émulsion de type huile-dans-l'eau (H/E), sous forme d'émulsion multiple, par exemple de type eau-dans-l'huile-dans-l'eau (E/H/E) ou huile-dans-l'eau-dans-l'huile (H/E/H).

14. Agent cosmétique selon l'une des revendications 1 à 13, **caractérisé en ce qu'**il contient, par rapport au poids de l'agent prêt à l'emploi, 0,005 à 10 % en poids, de préférence 0,1 à 5 % en poids, de manière davantage préférée 0,15 à 4 % en poids, de manière encore davantage préférée 0,2 à 3 % en poids, de manière particulièrement préférée 0,25 à 2 % en poids et en particulier 0,3 à 1,5 % en poids, de copolymères réticulés, partiellement ou totalement neutralisés, d'acide 2-méthyl-2[(1-oxo-2-propényl)amino]-1-propanesulfonique (AMPS) et d'acide acrylique.

15. Procédé cosmétique, non thérapeutique pour l'éclaircissement de la peau et/ou pour l'éclaircissement de lentigos séniles ou de taches de rousseur, **caractérisé en ce qu'**un agent cosmétique contenant
a) au moins un extrait d'Opuntia Ficus Indica ;
b) au moins un extrait de plantes de la famille des Brassicae ;
c) 2,3-dihydro-5,6-diméthoxy-3,3-diméthyl-2-(phénylméthyléno)-1H-indéno-1-one (benzylidène-diméthoxydiméthylindanone)
est appliqué sur la peau.
